Europäisches Patentamt

European Patent Office

Office européen des brevets

(19)

(11) Publication number: **0 417 953 A1**

# (12) EUROPEAN PATENT APPLICATION

(21) Application number: 90309617.0

(22) Date of filing: 03.09.90

(51) Int. Cl.5: **C12P 17/16, C12N 15/54**

The applicant has filed a statement in accordance with Rule 28 (4) EPC (issue of a sample only to an expert). Accession number(s) of the deposit(s): FERM BP-2500, BP-2501, BP-3034.

The microorganism(s) has (have) been deposited with Fermentation Research Institute under numbers FERM BP-2500, BP-2501, BP-3034.

(30) Priority: 04.09.89 JP 228846/89

(43) Date of publication of application:
20.03.91 Bulletin 91/12

(84) Designated Contracting States:
DE FR GB

(71) Applicant: KYOWA HAKKO KOGYO CO., LTD.
6-1, Ohte-Machi Itchome
Chiyoda-ku Tokyo(JP)

(72) Inventor: Fujio, Tatsuro
2-8-201, Kyowa-machi
Hofu-shi, Yamaguchi-ken(JP)
Inventor: Hayashi, Midori
4-5-14-607, Hon-cho
Nakano-ku, Tokyo(JP)
Inventor: Iida, Akihiro
4-17-9, Morino
Machida-shi, Tokyo(JP)
Inventor: Nishi, Tatsunari
1444, Saltair Avenue, Apartment House 106
Los Angeles, CA 90025(US)
Inventor: Hagihara, Takeshige
2-3-302, Kyowa-machi
Hofu-shi, Yamaguchi-ken(JP)

(74) Representative: Lambert, Hugh Richmond et al
D. YOUNG & CO. 10 Staple Inn
London, WC1V 7RD(GB)

(54) Process for producing thiamine phosphates.

(57) Thiamine phosphates such as thiamine monophosphate and thiamine pyrophosphate are produced by allowing thiamine to react with ATP in an aqueous medium in the presence of an enzyme source having enzyme activity to catalyze the formation of thiamine phosphates from thiamine and ATP, and recovering the formed thiamine phosphates from the reaction mixture.

EP 0 417 953 A1

## PROCESS FOR PRODUCING THIAMINE PHOSPHATES

The present invention relates to a process for producing thiamine phosphates such as thiamine monophosphate (hereinafter referred to as TMP) and thiamine pyrophosphate (hereinafter referred to as TPP). TPP, which is coenzyme of thiamine (vitamin $B_1$), and TMP, which is an intermediate metabolite of TPP, are important substances not only as raw materials of nutrient preparations and raw materials of various drugs but also as biochemical reagents.

Many methods are known for producing thiamine phosphates by chemical phosphorylation of thiamine [British Patent No. 687,673, Journal of the American Chemical Society, 60 , 2263 (1938), ibid., 63 , 1160 (1941), Japanese Published Examined Patent Application No. 14751/1974, etc.].

The methods by chemical synthesis has disadvantages that a mixture of various phosphoric acid esters other than the desired product is formed so that complicated steps for purification are required, yield is extremely poor, etc. Accordingly, it is desirable to develop a process for producing TMP and TPP in a high yield at low cost.

According to the present invention there is provided a process for producing thiamine phosphates which comprises allowing thiamine to react with adenosine-3-phosphate (ATP) in an aqueous medium in the presence of an enzyme source having enzyme activity to catalyze the formation of TMP and/or TPP from thiamine and ATP to form TMP and/or TPP, and recovering the formed TMP and/or TPP from the reaction mixture.

Various preferred features and embodiments of the invention will now be described by reference to the following accompanying drawings, in which:

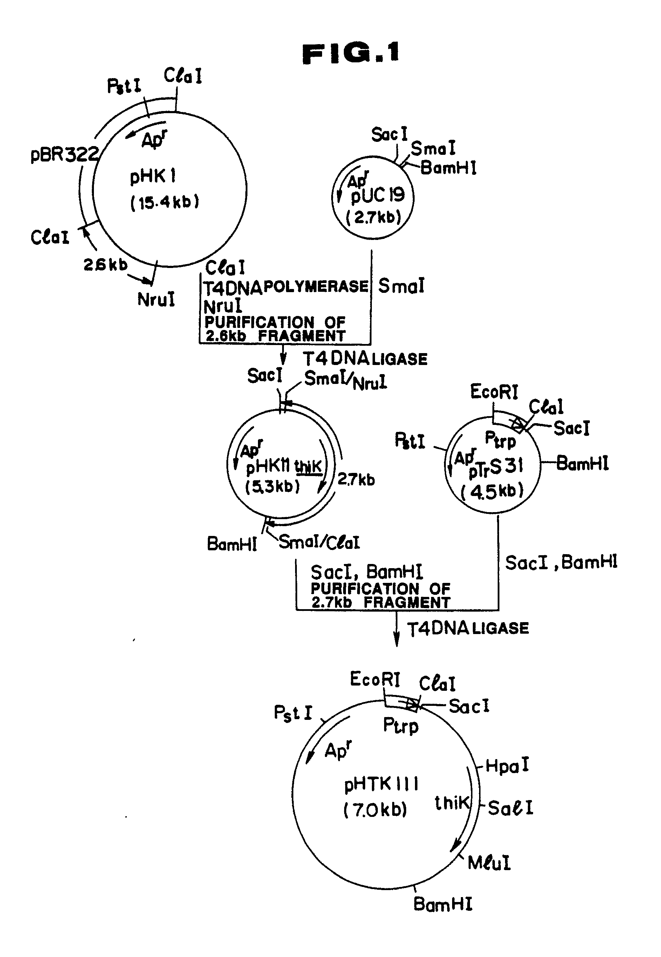

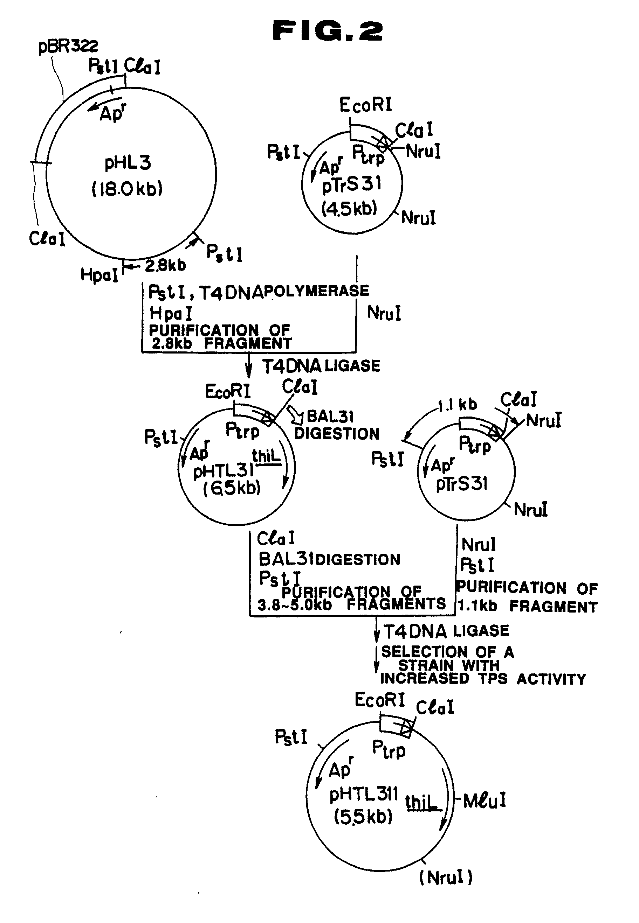

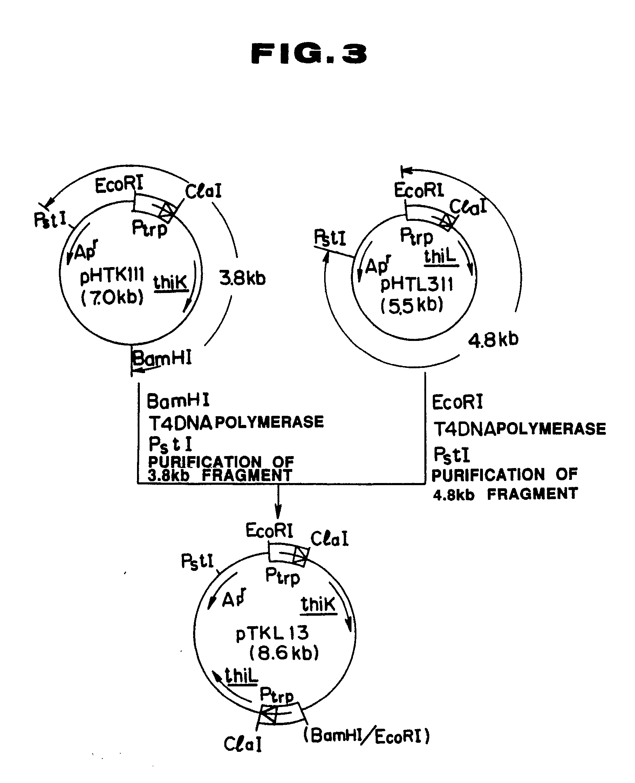

Figs. 1, 2, 3, 4 and 5 show steps of construction of plasmids pHTk111, pHTL311, pTKL13, pTrS31 and pHTL32, respectively.

Any enzyme source may be used in the present invention so long as it has enzyme activity to catalyze the formation of thiamine phosphates from thiamine and ATP, such as thiamine kinase activity or thiamine monophosphate kinase activity. Specifically, cells, culture and treated products of any microorganism capable of producing TMP and/or TPP from thiamine and ATP may be used.

An example of a suitable microorganism is Escherichia coli MM294 (ATCC 33625). Thiamine phosphates can be produced in a high yield by using a strain with an enhanced activity to produce thiamine phosphates from thiamine and ATP which carries an introduced recombinant DNA containing a DNA fragment bearing genetic information relating to the synthesis of TMP and/or TPP.

The recombinant DNA containing a DNA fragment bearing genetic information relating to the synthesis of TMP and/or TPP can be prepared by conventional recombinant DNA technique, using chromosomal DNA of a microorganism capable of producing thiamine phosphates from thiamine and ATP.

Examples of the DNA fragment bearing genetic information relating to the synthesis of TMP and TPP include DNA fragments containing a gene (hereinafter referred to as thiK) coding for thiamine kinase (EC2.7.1.89) (hereinafter referred to as TMK), a gene (hereinafter referred to as thiL) coding for thiamine monophosphate kinase (EC 2.7.4.16) (hereinafter referred to as TPS), etc.

As sources for supplying the DNA fragment containing a gene(s) coding for TMK and/or TPS, eucaryotes, procaryotes, bacteriophage, plasmids, etc. may be used, among which strains belonging to the genus Escherichia and having TMK and/or TPS activity are preferred. Examples of such strains are Escherichia coli W3110 (ATCC 27325) and Escherichia coli JA200 carrying Escherichia coli gene library constructed by Clarke, et al . [Cell, 9 , 91 (1976)]. The chromosomal DNA can be prepared by the method described in the EMBO Journal, 4 , 1875 (1985)].

As a vector for inserting the DNA fragment, any of phage vectors, plasmid vectors, etc. is usable as long as it is capable of autonomous replication in strains belonging to the genus Escherichia . Preferred examples are pBR322 [Gene, 2 , 95 (1977)] and pUC19 [Gene, 33 , 103 (1985)].

A recombinant DNA composed of a DNA fragment containing thiK and/or thiL and a vector DNA can be obtained as a mixture with various recombinant DNAs, by cleaving the chromosomal DNA of Escherichia coli as a source for supplying thiK and/or thiL and the vector DNA with an appropriate restriction enzyme such as Cla I, Pst I, or Hin dIII in vitro , respectively, and ligating both DNA fragments by the action of T4 DNA ligase.

The recombinant DNA containing a gene coding for the desired enzyme can be obtained by transforming a mutant defective in the gene by using the above mixture of recombinant DNAs, selecting a transformant in which the defectiveness is complemented, and isolating the recombinant DNA carried by the transformant. Escherichia coli NI510 (thiK1, thiD1) [J. Bacteriol., 151 , 708 (1982)] is an example of TMK-deficient mutant used for cloning of thiK, and Escherichia coli NI420 (thiB1, thiL2) [J. Bacteriol, 151 ,

708 (1982)] is an example of TPS-deficient mutant used for cloning of thiL. Transformation is carried out according to the method of Cohen, et al . [Cohen, et al ., Proc. Natl. Acad. Sci. U.S.A., 69 , 2110 (1979)].

After being isolated from the transformant, the recombinant DNA containing a DNA fragment bearing genetic information relating to the synthesis of TMP and/or TPP is introduced into a host microorganism, whereby a strain having an enhanced activity to produce thiamine phosphates from thiamine and ATP can be obtained.

Specific examples of such strains include Escherichia coli MH101 carrying a recombinant DNA in which thiK derived from Escherichia coli is incorporated, Escherichia coli MH301 and Escherichia coli MH302 carrying a recombinant DNA in which thiL derived from Escherichia coli is incorporated, and Escherichia coli MM294/pTKL13 carrying a recombinant DNA in which thiK and thiL derived from Escherichia coli are incorporated.

Cultivation of the microorganism capable of producing thiamine phosphates from thiamine and ATP is carried out by conventional methods for culturing bacteria. That is, the microorganism may be cultured in a conventional medium containing carbon sources, nitrogen sources, inorganic substances, amino acids, vitamins, etc., under aerobic conditions, at a controlled temperature, pH, etc.

Any carbon source may be used so long as it can be assimilated by the microorganism employed. Examples include various carbohydrates such as glucose, fructose, sucrose, molasses, blackstrap molasses and starch hydrolyzate; alcohols such as ethanol, glycerine and sorbitol; organic acids such as pyruvic acid, lactic acid and acetic acid; and amino acids such as glycine, alanine, glutamic acid and aspartic acid. It is preferable to use these carbon sources at a concentration of 5 to 30%.

Examples of nitrogen sources include ammonia, various inorganic and organic ammonium salts such as ammonium chloride, ammonium sulfate, ammonium nitrate, ammonium carbonate, ammonium acetate and ammonium phosphate; urea; nitrogen-containing organic substances such as peptone, NZ amine, meat extract, yeast extract, corn steep liquor, casein hydrolyzate, fish meal and its digested product; and various amino acids such as glycine and glutamic acid. These nitrogen sources are generally used at a concentration of 0.1 to 10%.

As the inorganic substances, potassium primary phosphate, potassium secondary phosphate, magnesium sulfate, magnesium phosphate, sodium chloride, ferrous sulfate, manganese sulfate, zinc sulfate, calcium carbonate, etc. may be used. In cases where the microorganism used requires particular nutrient substances such as amino acids, nucleic acids, and vitamins for the growth, these substances are added to the medium in appropriate amounts.

Cultivation is carried out under aerobic conditions, for example, by shaking culture or by aeration-stirring culture. The temperature for the cultivation is preferably between 20 and 40°C. The period for the cultivation is generally 1 to 72 hours. The pH of the medium is desirably maintained around neutrality with ammonia, urea, a sodium hydroxide solution, etc.

The thus obtained culture of the microorganism may be used for the reaction as it is. Alternatively, the culture may be treated in various ways and the resulting products may be used for the reaction. Examples of the treated products include a concentrate of the culture, dried culture, surfactant-and/or organic solvent-treated culture, bacteriolytic enzyme-treated culture, cells obtained by centrifugation of the culture, dried cells, acetone-treated cells, surfactant and/or organic solvent-treated cells, bacteriolytic enzyme-treated cells, immobilized cells, and enzyme preparation extracted from the cells.

The reaction for producing thiamine phosphates from thiamine and ATP can be carried out in any aqueous medium. Preferably, during cultivation of the microorganism or after completion of the cultivation, thiamine and ATP are added to the culture as substrates, if necessary, together with a surfactant and/or an organic solvent.

In cases where ATP used as a substrate is supplied from a microorganism capable of biosynthesizing ATP from a ATP precursor, a phosphoryl donor and an energy donor, the above reaction is carried out in the presence of the ATP precursor, the ATP-regenerating energy donor, the phosphoryl donor and the microorganism capable of biosynthesizing ATP instead of ATP.

The reaction mixture is incubated at 20 to 50°C for 1 to 48 hours, whereby TMP and/or TPP is accumulated in the reaction mixture. It is preferred to adjust the pH to 6 to 9 during the reaction.

Any of chemically synthesized thiamine, natural thiamine, pure thiamine and crude thiamine may be used for the reaction so long as it does not contain any inhibitor of the TMP- and TPP-producing reaction. A preferred concentration of thiamine is 0.1 to 50 g/ℓ.

In the production of TPP, TMP may be used as a substrate instead of thiamine. In this case, TMP may be any of the pure product and crude product so long as it does not contain any inhibitor of the TPP-producing reaction. A preferred concentration of TMP is 0.1 to 50 g/ℓ.

Any of pure ATP and crude ATP may be used so long as it does not contain any inhibitor of the reaction. When ATP is added as a substrate for the reaction, it is used generally in a concentration of 0.1 to 100 g/ℓ. On the other hand, when the reaction system coupled to the ATP biosynthesis system is used, addition of a catalytic amount (less than 1.0 g/ℓ of ATP is sufficient for the purpose. When the amount of ATP supplied from the cells or culture to the reaction system is sufficient for the needs, it is not necessary to add ATP.

The ATP precursor is usually supplied from the cells or culture to the reaction system in a sufficient amount. However, it may be added to the reaction mixture, if necessary. In this case, any pure or crude product of 5'-adenosine diphosphate, 5'-adenosine monophosphate, adenosine, adenine, etc. and their mixtures may be used so long as it can be converted into ATP by the microorganism used in the reaction and does not contain any inhibitor of the phosphorylation. Generally, use of the precursor in a concentration of less than 1 g/ℓ is sufficient.

As the microorganism capable of biosynthesizing ATP from the ATP precursor, the phosphoryl donor and the energy donor, any microorganism may be used so long as it belongs to the genus Esherichia , Brevibacterium or Corynebacterium and has the activity of ATP biosynthesis. Specific examples of the microorganism include Escherichia coli MM294 (ATCC 33625), Brevibacterium ammoniagenes ATCC 6872, and Corynebacterium glutamicum ATCC 13032.

As the ATP-regenerating energy donor, any of carbohydrates such as glucose, arabinose, fructose, lactose, maltose, sucrose, mannitol, sorbitol, trehalose, molasses, blackstrap molasses and other sugars, and starch hydrolyzate; organic acids such as pyruvic acid, lactic acid, acetic acid and $\alpha$-ketoglutaric acid; and amino acids such as glycine, alanine, aspartic acid, glutamic acid and glutamine may be used so long as it can be assimulated by the microorganism employed. In addition, phosphorylated compounds such as acetyl phosphate, carbamyl phosphate and creatin phosphate may be used. The energy donor is usually used at a concentration of 1 to 200 g/ℓ.

As the phosphoryl donor, any of sodium salts, potassium salts, magnesium salts, etc. of inorganic phosphoric acids such as orthophosphoric acid, pyrophosphoric acid, polyphosphoric acid and polymetaphosphoric acid may be used. Further, organic phosphorylated compounds such as acetyl phosphate, carbamyl phosphate, creatin phosphate and fructose-1,6-diphosphate may also be used. It is preferred to maintain its concentration in a range of 10 to 400 mM.

As the surfactant, cationic surfactants such as polyoxyethylene stearylamine (for example, NYMEEN S-215, manufactured by Nippon Oils and Fats Co., Ltd.; hereinafter the same shall apply), cetyl trimethyl ammonium bromide, cation FB and cation F2-40E; anionic surfactants such as sodium oleylamide sulfate, NEWREX TAB and RAPISOL 80; amphoteric surfactants such as polyoxyethylene sorbitan monostearate (for example, NONION ST221); tertiary amine PB, hexadecyldimethylamine, etc. may be used so long as it accelerates phosphorylation. These surfactants are used generally in a concentration of 0.1 to 50 mg/mℓ, preferably 1 to 20 mg/mℓ.

As the organic solvent, toluene, xylene, aliphatic alcohols, benzene, ethyl acetate, etc. are used in a concentration of 0.1 to 50 μℓ/mℓ, preferably 1 to 20 μℓ/mℓ.

TMP and/or TPP formed in the reaction mixture can be recovered by appropriate selection and combination of known ion exchange resin method, activated carbon adsorption method, extraction and precipitation with a solvent, and the like.

Various preferred embodiments of the invention are illustrated in the following representative examples.

Example 1

Construction of a recombinant plasmid in which a DNA fragment containing thiK gene derived from Escherichia coli is inserted:

(1) Cloning of thiK gene

Cloning of thiK gene was carried out using a strain having TMP-requirement due to thiK-defective mutation as a recipient. Introduction of the gene into the recipient was carried out by transfer of a plasmid to a recipient (conjugative transfer) on a selective agar plate accompanying transfer of F factor from $F^+$ donor to $F^-$ recipient, according to the replica mating method [L. Clarke, et al ., Methods in Enzymology, 68 , 396 (1979)]. As the DNA donor, JA200 strain ($F^+$) carrying gene library of Escherichia coli constructed by Clarke, et al . was used. As the recipient, TMP-requiring mutant NI510 (thiD, thiK, $F^-$) constructed by

Nakayama, et al . [H. Nakayama, et al ., J. Bacteriol., 151 , 708 (1982) ] was used. A strain showing resistance to streptomycin (hereinafter referred to as Stm) of the recipient and at the same time, showing TMP-non-requirement due to the introduction of the gene was selected.

First, Escherichia coli NI510 strain was cultured in L medium [medium containing 10 g/ℓ Bacto-tryptone (Difco Laboratories), 5 g/ℓ yeast extract (Difco Laboratories) and 5 g/ℓ NaCl; pH was adjusted to 7.2] containing 1 mg/ℓ TPP at 30° for 18 hours. Then, the cultured cells were thoroughly washed with physiological saline and appropriately diluted. The diluted culture was spread on MMStmB₁ plate medium for selection prepared by adding 50 mg/ℓ Stm, 1 mg/ℓ thiamine and 1.5% agar to MM medium [a medium prepared by dissolving 4 g of Na₂HPO₄, 2.5 g of KH₂PO₄, 1 g of (NH₄)₂SO₄, 0.1 g of MgSO₄·7H₂O, 5 mg of CaCl₂·2H₂O, 0.25 mg of FeSO₄·7H₂O, 25 mg of histidine, 25 mg of methionine, 25 mg of tryptophan and 50 mg of arginine in 1 liter of water; pH was not adjusted]. The donor strain was cultured on a plate medium obtained by adding 1.5% agar to L medium at 30°C for 18 hours, followed by replication on the selection medium on which the culture of the recipient was spread. After culturing on the plate medium at 30°C for 18 hours, a strain (NI510/pLC18-2) in which TMP-requirement of the recipient was complemented by introduction of a plasmid from the donor was selected.

Growth of NI510/pLC18-2 strain was examined on plate media. The strain grew on MMStmB₁ plate medium but did not grow on MMStm plate medium prepared by adding 50 mg/ℓ Stm and 1.5% agar to MM medium. From the results, it was confirmed that the plasmid did not complement the thiamine-requirement of NI510 strain but complemented TMP-requirement of NI510 strain. This suggests that thiK gene of Escherichia coli , not thiD gene, is present on pLC18-2.

(2) Subcloning of thiK on pBR322

Plasmid DNA was isolated from the selected strain and purified by a known method [Molecular Cloning: Cold Spring Harbor Lab., (1982); hereinafter this method was used to obtain plasmids]. In 50 μℓ of Y-50 buffer [10 mM Tris buffer (pH 7.5), 50 mM NaCl, 7 mM MgCl₂ and 1 mM dithiothreitol (hereinafter referred to as DTT)] was dissolved 1 μg of the DNA, and 5 units of restriction enzyme Cla I (Takara Shuzo Co., Ltd.; hereinafter restriction enzymes manufactured by Takara Shuzo Co., Ltd. were used unless otherwise indicated) was added to the solution. After digestion reaction at 37°C for 2 hours, the resulting digestion products were analyzed. The results reveal that the plasmid had a size of 28 kilobases (hereinafter referred to as kb) and had 4 Cla I cleavage sites.

A Cla I-cleaved fragment of pLC18-2 was cloned at the Cla I cleavage site of pBR322 in the following manner. In 50 μℓ of Y-50 buffer was dissolved 5 μg of pLC18-2 plasmid DNA, and 20 units of Cla I was added to the solution. Digestion reaction was carried out at 37°C for 2 hours and stopped by heat treatment at 65°C for 10 minutes. After addition of 130 μℓ of distilled water and 20 μℓ of 3 M sodium acetate (pH 5.6) to the digestion product, ice-cooled ethanol (twice volume of the mixture) was added to the mixture, and the resulting mixture was allowed to stand at -80°C for 20 minutes. Then, the mixture was subjected to centrifugation, and the supernatant was discarded to obtain DNA precipitates. (Hereinafter this method is referred to as the ethanol precipitation method.) Separately, 2 μg of pBR322 plasmid DNA (Takara Shuzo Co., Ltd.) was dissolved in 50 μℓ of Y-50 buffer, and 10 units of Cla I was added to the solution. Digestion reaction was carried out at 37°C for 2 hours and stopped by heat treatment at 65°C for 10 minutes. DNA precipitates were obtained by the ethanol precipitation method. About 0.2 μg of the Cla I-digested fragment of pLC18-2 and about 0.05 μg of the Cla I-digested fragment of pBR322 were dissolved in 50 μℓ of T4 ligase buffer [20 mM Tris-hydrochloride buffer (pH 7.6), 7 mM MgCl₂, 10 mM DTT and 0.5 mM ATP]. After 2 units of T4 DNA ligase (Takara Shuzo Co., Ltd.) was added to the solution, the mixture was allowed to stand at 16°C for 18 hours. The resulting recombinant DNA mixture was used for transformation of Escherichia coli NI510 strain by the method of Cohen, et al . [Proc. Natl. Acad. Sci. U.S.A., 69 , 2110 (1979)]. A transformant which grew on MMApB₁ plate medium obtained by adding 75 mg/ℓ ampicillin (hereinafter referred to as Ap) and 1 mg/ℓ thiamine to MM medium was selected. Plasmid DNA was isolated from the transformant and purified by a known method. By digesting the DNA with Cla I, the structure of the plasmid was analyzed. As the result, it was confirmed that it was a recombinant plasmid wherein the DNA fragment of about 11 kb derived from pLC18-2 was inserted at the Cla I cleavage site of pBR322. The plasmid was named pHK1. Escherichia coli MM294 (ATCC 33625) strain was transformed with pHK1 to obtain MM294/pHK1 strain.

(3) Subcloning of thiK

After pHK1 DNA was isolated and purified, the DNA was digested with various restriction enzymes to analyze its structure. pHK1 was cleaved with Cla I at two sites, with Pst I at three sites, with Sma I at one site and with NruI at at least two sites. Among the obtained fragments, a Cla I-Nru I-cleaved fragment of about 2.6 kb was expected to be the one on which thiK was present.

In 50 $\mu\ell$ of Y-50 buffer was dissolved 5 $\mu$g of pHK1 plasmid DNA prepared as described above, and 20 units of Cla I was added to the solution. Digestion reaction was carried out at 37°C for 2 hours. After heat treatment at 65°C for 10 minutes, DNA precipitates were obtained by the ethanol precipitation method. The DNA fragment was dissolved in 50 $\mu\ell$ (total volume) of T4 DNA polymerase buffer [67 mM Tris-hydrochloride buffer (pH 8.8), 6.7 mM MgCl$_2$, 6.6 mM (NH$_4$)$_2$SO$_4$, 10 mM 2-mercaptoethanol, 6.7 $\mu$M EDTA, 0.33 mM dCTP, 0.33 mM dATP, 0.33 mM dGTP and 0.33 mM dTTP]. After 5 units of T4 DNA polymerase (Takara Shuzo Co., Ltd.) was added to the solution, the mixture was incubated at 37°C for one hour, whereby the 5'-cohesive end formed by the digestion with Cla I was changed to the blunt end. To the mixture was added an equal amount of a mixture of phenol : chloroform (1 : 1 by volume), followed by adequate stirring to stop the reaction. The reaction mixture was centrifuged and the supernatant was obtained. (Hereinafter the procedure is referred to as the phenol : chloroform extraction method.) Then, DNA precipitates were obtained by the ethanol precipitation method. The DNA precipitates were dissolved in 50 $\mu$, of Nru I buffer [10 mM Tris-hydrochloride buffer (pH 7.5), 7 mM MgCl$_2$, 150 mM KCl, 1 mM DTT and 0.01% bovine serum albumin (hereinafter referred to as BSA)], and 20 units of Nru I was added to the solution. Digestion reaction was carried out at 37°C for 2 hours. After heat treatment at 65°C for 10 minutes, the Cla I-Nru I-cleaved fragment of about 2.6 kb was purified.

pUC19 isolated and purified from Escherichia coli JM109 strain [Gene, 33 , 103 (1985)] was used as the vector. In 50 $\mu\ell$ of a buffer composed of 10 mM Tris-hydrochloride buffer (pH 8.0), 7 mM MgCl$_2$, 1 mM DTT and 0.01% BSA was dissolved 2 $\mu$g of pUC19 DNA, and 20 units of Sma I was added to the solution. Digestion reaction was carried out at 37°C for 2 hours. After heat treatment at 65°C for 10 minutes, DNA precipitates were obtained by the ethanol precipitation method. About 0.2 $\mu$g of the DNA fragment of pHK1 and about 0.1 $\mu$g of the DNA fragment of pUC19 were subjected to ligation reaction in 50 $\mu\ell$ of T4 DNA ligase buffer at 16°C for 18 hours using 2 units of T4 DNA ligase. The thus obtained recombinant plasmid was used for transformation of Escherichia coli NI510 strain. Transformants which were resistant to Ap and in which TMP-requirement of the host was complemented were obtained by selecting strains which grew on MMApB$_1$ plate medium. Plasmid DNA was isolated from these transformants and purified. Its structure was analyzed by digestion with restriction enzymes, and as the result, it was confirmed that it was a plasmid wherein the DNA fragment of 2.6 kb was inserted. The plasmid was named pHK11. Escherichia coli MM294 strain was transformed with pHK11 to obtain Escherichia coli MM294/pHK11.

(4) Ligation of trp promoter (hereinafter referred to as Ptrp) in the upstream region of thiK

In 40 $\mu\ell$ of Y-0 buffer (a buffer having the composition of Y-50 buffer excluding NaCl) was dissolved 3 $\mu$g of pHK11 DNA obtained in (3), and 20 units of Sac I was added to the solution. Digestion reaction was carried out at 37°C for 2 hours. To the reaction mixture were added 2.5 $\mu\ell$ of 2 M NaCl and 15 units each of Bam HI and Sac I, followed by digestion reaction at 37°C for 2 hours. After heat treatment at 65°C for 10 minutes, a Sac I-Bam HI-cleaved DNA fragment of 2.7 kb containing thiK was purified.

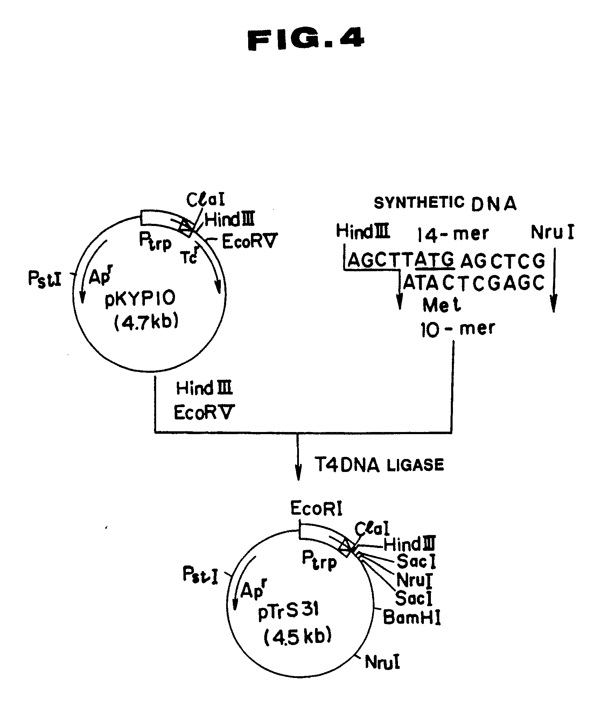

pTrS31 which is ATG-vector carrying Ptrp was used as the vector. Construction of pTrS31 is described in Reference Example. pTrS31 plasmid has 2 Sac I-cleavage sites and one Bam HI-cleavage site in the downstream region of Ptrp (Fig. 4). pTrS31 plasmid DNA (2 $\mu$g) isolated and purified was digested with Sac I and Bam HI in the same manner as above, and DNA precipitates were obtained by the ethanol precipitation method. About 0.2 $\mu$g of the DNA fragment of pHK11 and about 0.1 $\mu$g of the DNA fragment of pTrS31 were subjected to ligation reaction in 50 $\mu\ell$ of T4 DNA ligase buffer using 2 units of T4 DNA ligase. The ligation reaction was carried out at 16°C for 18 hours. The thus obtained recombinant plasmid was used for transformation of Escherichia coli MM294 strain to obtain Ap-resistant transformants. Plasmid DNA was isolated from the transformants and purified, and its structure was analyzed. As the result, it was confirmed that the DNA fragment of pHK11 containing thiK was inserted in the region downstream of Ptrp of pTrS31. The plasmid was named pHTK111 (Fig. 1). Escherichia coli MM294 strain was transformed with pHTK111 to obtain Escherichia coli MM294/pHTK111, which was deposited with the Fermentation Research Institute, Agency of Industrial Science and Technology, Japan, on July 3, 1989 as Escherichia coli MH101 (FERM BP-2500).

EP 0 417 953 A1

Example 2

Construction of a recombinant plasmid in which a DNA fragment containing thiL gene derived from Escherichia coli is inserted:

(1) Cloning of thiL gene

Cloning of thiL gene was carried out in the same manner as in Example 1 except that Escherichia coli NI420 (thiB, thiL, F⁻) which was TPP-requiring mutant [H. Nakayama, et al .; J. Bacteriol., 151 , 708 (1982)] was used as a recipient. A transformant (NI420/PLC30-6) showing resistance to Stm of the recipient and also showing TPP-non-requirement was obtained by selecting a strain which grew on MMStmB₁ selection plate medium. As NI420 strain could not grow on MMStmB₁ selection medium by introduction of thiB gene, it was considered that thiL gene of Escherichia coli , not thiB gene, was present on pLC30-6 and complemented TPP-requirement of NI420 strain.

(2) Subcloning of thiL on pBR322

Plasmid DNA was isolated from the selected strain and purified by a known method. Its structure was analyzed by cleavage with Cla I. The result revealed that the plasmid had a size of 20 kb and had 2 Cla I cleavage sites.

A Cla I-cleaved fragment of pLC30-6 was cloned at the Cla I cleavage site of pBR322 in the following manner. In 50 μℓ of Y-50 buffer was dissolved 5 μg of pLC30-6 plasmid DNA, and 20 units of Cla I was added to the solution. Digestion reaction was carried out at 37°C for 2 hours and stopped by heat treatment at 65°C for 10 minutes. DNA precipitates were obtained from the digestion product by the ethanol precipitation method. Separately, 2 μg of pBR322 plasmid DNA was dissolved in 50 μℓ of Y-50 buffer, and 10 units of Cla I was added to the solution. Digestion reaction was carried out at 37°C for 2 hours and stopped by heat treatment at 65°C for 10 minutes. DNA was obtained by the ethanol precipitation method. About 0.2 μg of the Cla I-digested fragment of pLC30-6 and about 0.05 μg of the Cla I-digested fragment of pBR322 were dissolved in 50 μℓ of T4 ligase buffer. After 2 units of T4 DNA ligase was added to the solution, the mixture was allowed to stand at 16°C for 18 hours. The resulting recombinant DNA mixture was used for transformation of Escherichia coli NI420 strain, and a transformant which grew on MMApB₁ plate medium was selected. Plasmid DNA was isolated from the transformant and purified by a known method. By digesting the DNA with Cla I, the structure of the plasmid was analyzed. As the result, it was confirmed that it was a recombinant plasmid wherein the DNA fragment of about 14 kb derived from pLC30-6 was inserted at the Cla I cleavage site of pBR322. The plasmid was named pHL3. Escherichia coli MM294 strain was transformed with pHL3 to obtain MM294/pHL3 strain.

(3) Ligation of trp promoter in the upstream region of thiL

After pHL3 DNA was isolated and purified, the DNA was digested with various restriction enzymes to analyze its structure. pHL3 was cleaved with Pst I at four sites, with Hin dIII at three sites and with Hpa I at two sites. Among the obtained fragments, a Pst I-Hpa I-cleaved fragment of about 2.8 kb was expected to be the one on which thiL was present.

In 50 μℓ of Y-50 buffer was dissolved 5 μg of pHL3 plasmid DNA prepared as described above, and 20 units of Pst I was added to the solution. Digestion reaction was carried out at 37°C for 2 hours. After heat treatment at 65°C for 10 minutes, DNA precipitates were obtained by the ethanol precipitation method. The DNA fragment was dissolved in 50 μℓ (total volume) of T4 DNA polymerase buffer. After 5 units of T4 DNA polymerase was added to the solution, the mixture was incubated at 37°C for one hour, whereby the 3'-cohesive end formed by the digestion with Pst I was changed to the blunt end. The mixture was treated by the phenol:chloroform extraction method, and then DNA precipitates were obtained by the ethanol precipitation method. The DNA precipitates were dissolved in 50 μℓ of a buffer composed of 10 mM Tris-hydrochloride buffer (pH 7.5), 7 mM MgCl₂, 100 mM KCl, 1 mM DTT and 0.01% BSA, and 20 units of Hpa I was added to the solution. Digestion reaction was carried out at 37°C for 2 hours. After heat treatment at 65°C for 10 minutes, the Pst I-Hpa I-cleaved fragment of about 2.8 kb was purified.

7

pTrS31 was used as the vector. In 50 $\mu\ell$ of Nru I buffer was dissolved 2 $\mu$g of pTrS31 plasmid DNA isolated and purified, and 15 units of Nru I was added to the solution. Digestion reaction was carried out at 37°C for 2 hours. After heat treatment at 65°C for 10 minutes, DNA precipitates were obtained by the ethanol precipitation method. About 0.2 $\mu$g of the DNA fragment (2.8 kb) of PHL3 and about 0.1 $\mu$g of the DNA of pTrS3I were subjected to ligation reaction in 50 $\mu\ell$ of T4 DNA ligase buffer at 16°C for 18 hours, using 2 units of T4 DNA ligase. The thus obtained recombinant plasmid was used for transformation of Escherichia coli MM294 strain to obtain Ap-resistant transformants.

Plasmid DNA was isolated from these transformants and purified, and its structure was analyzed by digestion with various restriction enzymes. As the result, it was confirmed that the plasmid had a structure wherein the DNA fragment of pHL3 containing thiL was inserted in the region downstream of Ptrp of pTrS3I. The plasmid was named pHTL31.

(4) Shortening of the distance between Ptrp and thiL

As described below, the transformant carrying pHTL31 had a slightly increased TMP kinase activity compared with the transformant carrying pHL3. However, as extra intervening sequence seemed to be present in the upstream region of thiL on the fragment inserted in pHTL31, plasmid pHTL311 in which the distance between Ptrp and thiL was shortened was constructed by the following procedure.

About 10 $\mu$g of pHTL31 DNA was dissolved in 60 $\mu\ell$ of Y-50 buffer, and 40 units of Cla I was added to the solution. Digestion reaction was carried out at 37°C for 2 hours. To 30 $\mu\ell$ of the Cla I digestion reaction mixture were added 20 $\mu\ell$ of BAL31 buffer at 5-fold concentration [100 mM Tris-hydrochloride buffer (pH 8.1), 60 mM MgCl$_2$ 60 mM CaCl$_2$ and 3 M NaCl], 40 $\mu\ell$ of distilled water and 1.5 units of BAL31 (Takara Shuzo Co., Ltd.), and digestion reaction was carried out at 30°C. Five to 10 minutes after the initiation of the reaction, 10 $\mu\ell$ portions of the reaction mixture were taken at intervals and respectively added to 20 $\mu\ell$ of a mixture of phenol : chloroform (1 : 1 by volume). Each of the resulting mixtures was stirred well to terminate the reaction. After centrifugation, the supernatant was separated and ice-cooled ethanol (twice volume of the supernatant) was added thereto. The resulting mixture was allowed to stand at -80°C for 30 minutes. After centrifugation, the supernatant was discarded and the precipitates were dissolved in 50 $\mu$, of Y-50 buffer. To each of the solutions was added 3 units of Pst I, and digestion reaction was carried out at 37°C for 2 hours. After heat treatment of the reaction mixtures at 65°C for 10 minutes, fragments of 3.8 to 5.0 kb were purified.

pTrS31 was used as the vector. In 50 $\mu\ell$ of Nru I buffer was dissolved 3 $\mu$g of pTrS31 DNA, and 15 units of Nru I was added to the solution. Digestion reaction was carried out at 37°C for 2 hours. After the reaction, DNA fragment was obtained by the ethanol precipitation method. The resulting DNA fragment was dissolved in 50 $\mu\ell$ of Y-50 buffer, and 15 units of Pst I was added to the solution. Digestion reaction was carried out at 37°C for 2 hours. From the digestion product, a DNA fragment of about 1.1 kb containing Ptrp was purified.

Then, the DNA fragments of pHTL31 (0.1 $\mu$g each) were respectively dissolved in 20 $\mu\ell$ (total volume) of T4 DNA ligase buffer and 0.05 $\mu$g of the DNA fragment of pTrS31 was added to each solution, followed by addition of one unit of T4 DNA ligase. Ligation reaction was carried out at 16°C for 18 hours. The resulting recombinant plasmid DNA was used for transformation of Escherichia coli MM294 strain and Ap-resistant transformants were selected. The Ap-resistant strains were cultured in M9Ap liquid medium obtained by adding 100 mg/$\ell$ Ap to M9 liquid medium (a medium containing 1 g of NH$_4$Cl, 6 g of Na$_2$HPO$_4$, 3 g of KH$_2$PO$_4$, 5 g of NaCl, 0.25 g of MgSO$_4$•7H$_2$O, 3 g of glucose, 4 mg of thiamine and 2 g of Casamino acid in 1 liter of water) at 30°C for 18 hours. TMP kinase activity was examined by the method described below, and a strain having activity higher than that of MM294 strain carrying pHTL31 was selected. The activity of the thus obtained strain was approximately 1.8 times higher than that of MM294/pHTL31 strain. The plasmid DNA named pHTL311 was isolated and purified from the strain, and its structure was analyzed. The results revealed that pHTL311 had a structure in which the distance between Ptrp and thiL on the fragment inserted downstream thereof was shortened by about 1 kb compared with pHTL31. Escherichia coli MM294 strain was transformed with pHTL311 to obtain Escherichia coli MM294/pHTL3II. The strain was deposited with the Fermentation Research Institute, Agency of Industrial Science and Technology, Japan, on July 3, 1989 as Escherichia coli MH301 (FERM BP-2501).

(5) Construction of plasmid pHL32 capable of expressing thiL gene with high efficiency:

8

DNA nucleotide sequence around the N-terminal of thiL structural gene of plasmid pHTL311 was determined by the Maxam-Gilbert method [A.M. Maxam, et al ., Proc. Natl. Acad. Sci., 74 , 560 (1977)]. The result revealed that there was still extra sequence of about 500 bp between the SD sequence of Ptrp and the initiation codon of the thiL gene. It was also found that a sequence presumed to take a terminator-like secondary structure was present just upstream of the thiL gene. It was considered that this sequence would inhibit high expression of thiL. The analysis of the DNA nucleotide sequence further revealed that Nsp -(7524)I cleavage site was present just after the initiation codon of the thiL gene as shown below.

```
                                  Nsp( 7524) I
                                       ┬
    ...GAGGCATAACGTATGGCATG|TGGC...
                                  ┌
                     MetAlaCysGly...
```

Therefore, the following linker was synthesized with the purpose of removing the extra sequence using this Nsp (7524)I cleavage site and ligating the thiL structural gene at a suitable distance from the SD sequence of Ptrp.

```
    ClaI                     Nsp( 7524 )I
     |CGATAAGCTTATGGCATG|        (18-mer)
     |TATTCGAATACC|              (12-mer)
     |             Met |
```

First, single-stranded DNAs of 18-mer and 12-mer were synthesized by the conventional triester method [R. Crea, et al .: Proc. Natl. Acad. Sci., 75 , 5765 (1978)]. Then, 2 µg each of the 18-mer and 12-mer single-stranded DNAs were dissolved in 20 µ$\ell$ (total volume) of a solution containing 50 mM Tris-hydrochloride buffer (pH 7.5), 10 mM $MgCl_2$, 5 mM DTT, 0.1 mM EDTA and 1 mM ATP. Thirty units of T4 polynucleotide kinase (Boehringer Mannheim GmbH) was added to the solution, and phosphorylation reaction was carried out at 37° C for 60 minutes. The phosphorylated 18-mer and 12-mer DNAs (2 µg each) were mixed, and the mixture was heated at 70° C for 5 minutes and allowed to stand at room temperature to effect annealing, whereby the DNA linker having the structure shown above was obtained.

In 50 µ$\ell$ of K-100 buffer [20 mM Tris-hydrochloride buffer (pH 8.5), 10 mM $MgCl_2$, 1 mM DTT and 100 mM KC1] was dissolved 3 µg of pTrS31 plasmid DNA, and 15 units each of restriction enzymes Cla I and Eco T14I were added to the solution. Digestion reaction was carried out at 37° C for 2 hours. After heat treatment at 65° C for 10 minutes, a DNA fragment of about 3.4 kb was purified.

In 50 µ$\ell$ of K-100 buffer containing 0.01% BSA was dissolved 3 µg of pHTL311 plasmid DNA, and 15 units each of restriction enzymes Nsp (7524)I and Eco T14I were added to the solution. Digestion reaction was carried out at 37° C for 2 hours. After heat treatment at 65° C for 10 minutes, a DNA fragment of about 1.6 kb was purified.

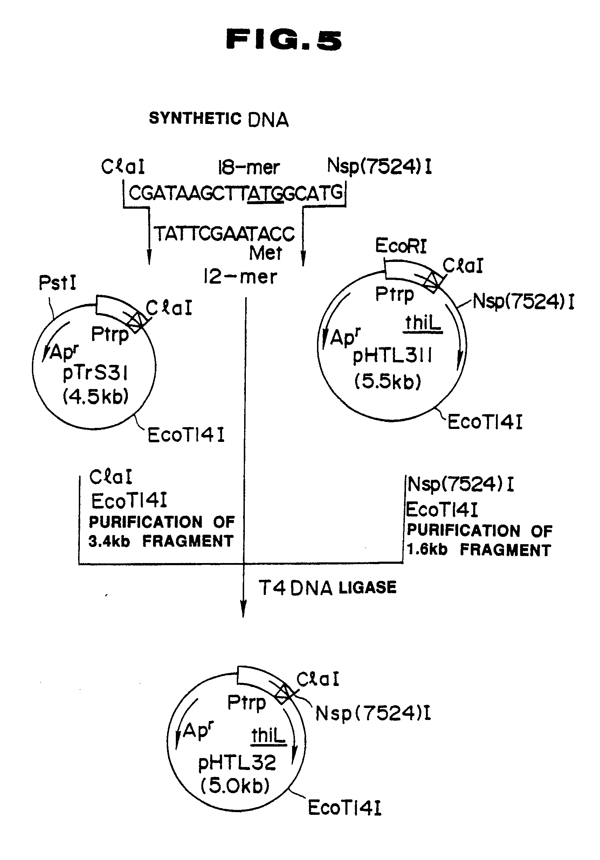

Then, 0.5 µg each of the Cla I-Eco T14I-digested DNA fragment of 3.4 kb of pTrS31 and the Nsp -(7524)I-Eco T14I-digested DNA fragment of 1.6 kb of pHTL311 were dissolved in 25 µ$\ell$ of T4 DNA ligase buffer, followed by addition of about 0.1 µg of the DNA linker described above. To the resulting mixture was added 4 units of T4 DNA ligase, and ligation reaction was carried out at 16° C for 18 hours. The thus obtained recombinant plasmid was used for transformation of Escherichia coli MM294 strain according to a conventional method. Plasmid was isolated from the resulting Ap-resistant transformant, and named pHTL32 (Fig. 5). The nucleotide sequence around the initiation codon (ATG) of the thiL gene of plasmid pHTL32 was confirmed by the method of Maxam-Gilbert, et al ., which is shown below.

```
          ClaI                   Nsp( 7524) I
                ┬                      ┬
  ...|AAGG|GTAT|CGATAAGCTTATGG|CATG|TGGC...
        SD       ┌            MetAlaCysGly...
```

pHTL32 is a plasmid capable of expressing thiL with high efficiency and having a structure in which the initiation codon of the thiL structural gene is ligated downstream of the sequence of Ptrp at a distance of 14 bp by using a linker.

After MM294/pHTL32 strain was cultured in M9Ap liquid medium at 30°C for 16 hours, it was examined for TPS activity by the method described below. The activity was about 15 times higher than that of MM294/pHTL3l strain.

The strain was deposited with the Fermentation Research Institute, Agency of Industrial Science and Technology, Japan, on July 31, 1990 as Escherichia coli MH302 (FERM BP-3034).

Example 3

Construction of a recombinant plasmid containing thiK and thiL derived from Escherichia coli :

A recombinant plasmid containing both thik and thiL derived from Escherichia coli was constructed using pHTK111 as a plasmid containing thik of Escherichia coli and pHTL311 as a plasmid containing thiL of Escherichia coli .

pHTK111 and pHTL311 plasmid DNAs were isolated and purified, respectively. In 50 $\mu\ell$ of Y-100 buffer (a buffer having the same composition as Y-50 buffer except that NaCl concentration is 100 mM) was dissolved 3 $\mu$g of pHTK111 DNA, and 20 units of Bam HI was added to the solution. Digestion reaction was carried out at 37°C for 2 hours. After heat treatment at 65°C for 10 minutes, DNA precipitates were obtained by the ethanol precipitation method. The DNA fragment was subjected to treatment with T4 DNA polymerase as in Example 1 (2) to change the Bam HI end of the DNA fragment to the blunt end, followed by extraction with phenol : chloroform. By the ethanol precipitation method, DNA fragment was obtained. The DNA fragment was dissolved in 50 $\mu\ell$ of Y-100 buffer, and digestion reaction was carried out at 37°C for 2 hours using 20 units of Pst I. Then, a fragment having a size of 3.8 kb was purified.

In 50 $\mu\ell$ of Y-50 buffer supplemented with 90 mM Tris-hydrochloride buffer (pH 7.5) wad dissolved 3 $\mu$g of pHTL311 DNA, and 20 units of Eco RI was added to the solution. Digestion reaction was carried out at 37°C for 2 hours. After heat treatment at 65°C for 10 minutes, DNA precipitates were obtained by the ethanol precipitation method. The DNA fragment was subjected to treatment with T4 DNA polymerase as in Example 1 (2) to change the end of the DNA fragment to the blunt end, followed by extraction with phenol : chloroform. By the ethanol precipitation method, DNA fragment was obtained. The DNA fragment was dissolved in 50 $\mu\ell$ of Y-100 buffer, and digestion reaction was carried out at 37°C for 2 hours using 20 units of Pst I. Then, a fragment having a size of 4.8 kb was purified.

Two units of T4 DNA ligase was added to 20 $\mu\ell$ (total volume) of T4 DNA ligase buffer containing 0.1 $\mu$g of the DNA fragment of pHTk111 and 0.1 $\mu$g of the DNA fragment of pHTL311, and ligation reaction was carried out at 16°C for 18 hours. The resulting recombinant plasmid DNA was used for transformation of Escherichia coli NI512 strain (thiD1, thiK1, thiL1) [J. Bacteriol., 151 , 708 (1982)]. An Ap-resistant transformant capable of growing on MMApB₁ plate medium was selected as a strain having both thiK and thiL genes. Plasmid DNA was isolated from the obtained transformant and purified. The result of structure analysis revealed that it was a recombinant plasmid in which the fragment containing thiK was ligated with the fragment containing thiL. The plasmid was named pTKL13. Escherichia coli MM294 strain was transformed with pTKL13 to obtain Escherichia coli MM294/pTKL13.

Example 4

Determination of TMK activity of a strain carrying a recombinant plasmid containing thiK gene:

The TMK activity was determined by a slight modification of the known method [J. Bacteriol., 112 , 1118 (1972)] as described below.

Escherichia coli strains carrying plasmids to be examined for the activity were inoculated into M9Ap liquid medium, respectively. Escherichia coli MM294 strain was inoculated into M9 liquid medium. After shaking culture at 30°C for 18 hours, the culture was centrifuged, if necessary after being diluted with TMK buffer [20 mM potassium phosphate buffer (hereinafter referred to as PK buffer) (pH 7.2), 1 mM MgCl₂ and 1 mM 2-mercaptoethanol]. The cells were collected and suspended in an appropriate amount of TMK buffer. After freezing at -20°C, the frozen cells were thawed and provided for reaction.

The frozen and thawed cells were added to a reaction solution composed of 100 mM PK buffer (pH 7.2), 10 mM $MgCl_2$, 10 mM ATP, 1 mM thiamine and 10 mℓ/ℓ xylene. The mixture was allowed to stand at 37°C to effect the reaction for converting thiamine into TMP.

The formation of TMP was determined in the following manner. That is, the reaction mixture was taken at intervals and adjusted to pH 4.5 with 0.2N hydrochloric acid. After heat treatment at 90°C for 5 minutes, the mixture was centrifuged and TMP in the supernatant was quantitatively determined by high performance liquid chromatography according to the known method [Analytical Biochemistry, 97, 191 (1979)].

TMK activity of the strains used or obtained in the present invention is shown in Table 1. The activity is indicated as a unit, one unit being defined as that amount of the enzyme capable of producing 1 μmol of TMP in one minute.

Table 1

| Strain | Activity (unit/g wet cell) | Relative Activity |
|---|---|---|
| MM294 | 0.0057 | 1.0 |
| MM294/pHK1 | 0.0577 | 10.1 |
| MM294/pHK11 | 0.0901 | 15.8 |
| MH101 (MM294/pHTK111) | 0.2066 | 36.2 |
| MM294/pTKL13 | 0.3749 | 65.8 |

Example 5

Determination of TPS activity of a strain carrying a recombinant plasmid containing thiL gene:

The TPS activity was determined in the following manner. Cultivation of Escherichia coli strains to be examined for the activity was carried out in a similar manner as in Example 4.

The resulting culture was centrifuged, if necessary after being diluted with TPS buffer [10 mM Tris-hydrochloride buffer (pH 7.5), 1 mM $MgCl_2$ and 1 mM 2-mercaptoethanol]. The cells were collected and suspended in an appropriate amount of TPS buffer. After freezing at -20°C, the frozen cells were thawed and provided for reaction.

The frozen and thawed cells were added to a reaction solution composed of 50 mM Tris-hydrochloride buffer (pH 7.5), 5 mM $MgCl_2$, 5 mM ATP, 337 mM KC1, 1 mM TMP and 10 mℓ/ℓ xylene. The mixture was allowed to stand at 37°C to effect the reaction for converting TMP into TPP.

The formation of TPP was determined in a similar manner as in the determination of TMP in Example 4.

TPS activity of the strains used or obtained in the present invention is shown in Table 2. The activity is indicated as a unit, one unit being defined as that amount of the enzyme capable of producing 1 μmol of TPP in one minute.

Table 2

| Strain | Activity (unit/g wet cell) | Relative Activity |
|---|---|---|
| MM294 | 0.0022 | 1.0 |
| MM294/pHL3 | 0.0033 | 1.5 |
| MM294/pHTL31 | 0.0104 | 4.7 |
| MH301 (MM294/pHTL311) | 0.0192 | 8.7 |
| MM294/pTKL13 | 0.0509 | 23.1 |
| MH302 (MM294/pHTL32) | 0.2090 | 122.3 |

Example 6

Production of TMP from thiamine using cells of Escherichia coli MH101 (FERM BP-2500) as enzyme source:

To 5 mℓ of 100 mM PK buffer were added 100 g (wet cell weight)/ℓ frozen cells of Escherichia coli MH101 strain cultured in a similar manner as in Example 4, 5 mM thiamine, 10 mM ATP, 10 mM MgCl₂ and 10 mℓ/ℓ xylene. The mixture was kept at 37°C for 3 hours, whereby 4.6 mM TMP was produced in the reaction mixture. As a control, the same reaction was carried out using Escherichia coli MM294 as the enzyme source. In this case, 0.2 mM TMP was produced.

Example 7

Production of TPP from TMP using cells of Escherichia coli MH301 (FERM BP-2501) as enzyme source:

To 5 mℓ of 50 mM Tris-hydrochloride buffer (pH 7.5) were added 100 g (wet cell weight)/ℓ frozen cells of Escherichia coli MH301 strain cultured in a similar manner as in Example 4, 5 mM TMP, 5 mM ATP, 337 mM KC1, 5 mM MgCl₂ and 10 mℓ/ℓ xylene. The mixture was kept at 37°C for 24 hours, whereby 1.6 mM TPP was produced in the reaction mixture. As a control, the same reaction was carried out using Escherichia coli MM294 as the enzyme source. In this case, 0.1 mM TPP was produced.

Example 8

Production of TPP from TMP using cells of Escherichia coli MH302 (FERM BP-3034) as enzyme source:

To 5 mℓ of 50 mM Tris-hydrochloride buffer (pH 7.5) were added 100 g (wet cell weight)/ℓ frozen cells of Escherichia coli MH302 strain cultured in a similar manner as in Example 4, 10 mM TMP, 5 mM ATP, 337 mM KC1 and 10 mℓ/ℓ xylene. The mixture was kept at 37°C for 3 hours, whereby 3.5 mM TPP was produced in the reaction mixture. As a control, the same reaction was carried out using Escherichia coli MM294 as the enzyme source. In this case, 0.07 mM TPP was produced.

Example 9

Production of TPP from thiamine using cells of a strain carrying a plasmid containing thiK and a strain carrying a plasmid containing thiL as enzyme sources:

To 5 mℓ of 100 mM PK buffer were added 100 g/(wet cell weight)/ℓ frozen cells of each of Escherichia coli MH101 strain and Escherichia coli MH302 strain cultured in a similar manner as in Example 4, 20 mM thiamine, 10 mM ATP and 10 mℓ/ℓ xylene. The mixture was kept at 37°C for 8 hours, whereby 5.0 mM TMP and 2.5 mM TPP were produced in the reaction mixture. As a control, the same reaction was carried out using Escherichia coli MM294 as the enzyme source. In this case, 0.1 mM TMP and 0.06 mM TPP were produced.

Example 10

Production of TPP from thiamine using cells of a strain carrying a recombinant plasmid containing thiK and thiL:

Cultivation of MM294/pTKL13 strain used in the reaction and treatment of the cells were carried out in a similar manner as in Example 4.

To 5 mℓ of a reaction solution composed of 100 mM PK buffer (pH 7.2), 10 mM MgCl₂, 5 mM ATP, 5 mM thiamine and 10 mℓ/ℓ xylene was added 100 g (wet cell weight)/ℓ frozen cells of MM294/PTKL13 strain to effect the reaction for converting thiamine into TMP and TPP. By the reaction at 37°C for 6 hours, 3.2 mM TMP and 0.6 mM TPP were produced in the reaction mixture. When the host strain MM294 was used instead of MM294/PTKL13 strain, 0.09 mM TMP and 0.02 mM TPP were produced.

Example 11

The reaction was carried out in a similar manner as in Example 6 except that 50 g/ℓ glucose and 10 g/ℓ Na₂HPO₄ were added in place of ATP and the reaction was carried out with shaking, during which the pH of the reaction mixture was adjusted to about 7.2 by adding 6 N KOH at intervals. By the reaction for 12 hours, 1.0 mM TMP was produced in the reaction mixture. As a control, the same reaction was carried out using Escherichia coli MM294 strain, whereby 0.11 mM TMP was produced.

Example 12

The reaction was carried out in a similar manner as in Example 7 except that 50 g/ℓ glucose and 10 g/ℓ Na₂HPO₄ were added in place of ATP and the reaction was carried out with shaking, during which the pH of the reaction mixture was adjusted to about 7.2 by adding 6 N KOH at intervals. By the reaction for 24 hours, 1.3 mM TPP was produced in the reaction mixture. As a control, the same reaction was carried out using Escherichia coli MM294 strain, whereby 0.14 mM TPP was produced.

Example 13

The reaction was carried out in a similar manner as in Example 10 except that 50 g/ℓ glucose and 10 g/ℓ Na₂HPO₄ were added in place of ATP and the reaction was carried out with shaking, during which the pH of the reaction mixture was adjusted to about 7.2 by adding 6 N KOH at intervals. By the reaction for 24 hours, 3.5 mM TMP and 1.1 mM TPP were produced in the reaction mixture. As a control, the same reaction was carried out using Escherichia coli MM294 strain, whereby 0.1 mM TMP and 0.11 mM TPP were produced.

Example 14

The reaction was carried out in a similar manner as in Example 8 except that 50 g/ℓ glucose and 10 g/ℓ Na₂HPO₄ were added in place of ATP and the reaction was carried out with shaking, during which the pH of the reaction mixture was adjusted to about 7.2 by adding 6 N KOH at intervals. By the reaction for 8 hours, 6.2 mM TPP was produced. As a control, the same reaction was carried out using Escherichia coli MM294 as the enzyme source. In this case, 0.07 mM TPP was produced.

Example 15

The reaction was carried out in a similar manner as in Example 9 except that 50 g/ℓ glucose and 10 g/ℓ Na₂HPO₄ were added in place of ATP and the reaction was carried out with shaking, during which the pH of the reaction mixture was adjusted to about 7.2 by adding 6 N KOH at intervals. By the reaction for 8 hours, 4.6 mM TMP and 3.9 mM TPP were produced. As a control, the same reaction was carried out using Escherichia coli MM294 as the enzyme source. In this case, 0.11 mM TMP and 0.05 mM TPP were produced.

Reference Example

EP 0 417 953 A1

Construction of Ptrp-ATG vector pTrS31:

Expression vector plasmid pKYP10 containing Ptrp (Japanese Published Unexamined Patent Application No. 110600/1983) has cleavage sites for Hin dIII and Eco RV in the region downstream of Ptrp. In 50 $\mu\ell$ of Y-50 buffer was dissolved 3 $\mu$g of pKYP10 plasmid DNA, and 15 units each of Hin dIII and Eco RV were added to the solution. Digestion reaction was carried out at 37°C for 2 hours. After heat treatment at 65°C for 10 minutes, DNA precipitates were obtained by the ethanol precipitation method.

Separately, the following DNA linker was synthesized with the purpose of constructing an expression vector (ATG vector) containing Ptrp and SD sequence at an appropriate distance from the translation initiation codon ATG, which is immediately followed by recognition sites for restriction enzymes ( Sph I, Sac I, etc.) which can be cleaved to form 3'-cohesive ends.

```
    HindIII          Met       SacI   NruI
    5' A G C T T A T G A G C T C G 3'      (14-mer)
          3' A T A C T C G A G C 5'        (10-mer)
```

First, single-stranded DNAs of 14-mer and 10-mer were synthesized by the conventional triester method [R. Crea, et al .: Proc. Natl. Acad. Sci., 75 , 5765 (1978)]. Then, 2 $\mu$g each of the 14-mer and 10-mer single-stranded DNAs were dissolved in 20 $\mu\ell$ (total volume) of a solution containing 50 mM Tris-hydrochloride buffer (pH 7.5), 10 mM MgCl$_2$, 5 mM DTT, 0.1 mM EDTA and 1 mM ATP. Thirty units of T4 polynucleotide kinase (Boehringer Mannheim GmbH) was added to the solution, and phosphorylation reaction was carried out at 37°C for 60 minutes.

The phosphorylated 14-mer and 10-mer DNAs (2 $\mu$g each) were mixed, and the mixture was heated at 70°C for 5 minutes and allowed to stand at room temperature to effect annealing, whereby the DNA linker having the structure shown above was obtained.

In 25 $\mu\ell$ (total volume) of T4 DNA ligase buffer was dissolved 0.5 $\mu$g of the Hin dIII-Eco RV-digested DNA fragment of pKYP10 obtained above, followed by addition of about 0.1 $\mu$g of the DNA linker. To the resulting mixture was added 4 units of T4 DNA ligase, and ligation reaction was carried out at 16°C for 18 hours. The thus obtained recombinant plasmid was used for transformation of Escherichia coli MM294 strain in a conventional manner and plasmid was isolated from the Ap-resistant transformant. The result of analysis of its structure revealed that the plasmid was pTrS31 shown in Fig. 4. The nucleotide sequence from the SD sequence (AAGG) to the region around the initiation codon (ATG) of plasmid pTrS31 was confirmed by the Maxam-Gilbert method [A. M. Maxam, et al ., Proc. Natl. Acad. Sci., 74 , 560 (1977)], which is shown below.

```
           ClaI HindIII       SacI NruI SacI (EcoRV)
    ...AAGGGTATCGATAAGCTTATGAGCTCGCGAGCTCATAATCG...
        SD                              Met
```

It was revealed that pTrS31 was ATG-vector having a structure in which the distance between the SD sequence and ATG was 14 bp and two Sac I cleavage sites and one Nru I cleavage site were present just after the ATG codon and was a plasmid in which two synthetic DNAs were inserted.

## Claims

1. A process for producing thiamine monophosphate and/or thiamine pyrophosphate which comprises reacting thiamine with adenosine-3-phosphate (ATP) in an aqueous medium in the presence of an enzyme source having enzyme activity to catalyze the formation of thiamine monophosphate and/or thiamine pyrophosphate from thiamine and ATP, and recovering the formed thiamine monophosphate and/or thiamine pyrophosphate from the reaction mixture.

2. A process according to claim 1, wherein said ATP is ATP supplied from a microorganism capable of biosynthesizing ATP from an ATP precursor, a phosphoryl donor and an energy donor.

14

EP 0 417 953 A1

3. A process according to claim 2, wherein said microorganism is a microorganism belonging to the genus Escherichia , Brevibacterium or Corynebacterium .

4. A process according to claim 1, wherein said enzyme source is thiamine kinase and/or thiamine monophosphate kinase.

5. A process according to claim 1, wherein said enzyme source is cells, culture or treated products of a microorganism capable of producing thiamine monophosphate and/or thiamine pyrophosphate from thiamine and ATP.

6. A process accoding to claim 5, wherein microorganism is a microorganism belonging to the genus Esherichia .

7. A process according to either of claims 5 and 6, wherein said microorganism is a microorganism which carries a recombinant DNA containing a DNA fragment bearing genetic information relating to the synthesis of thiamine phosphate and/or thiamine pyrophosphate.

8. A process according to claim 7, wherein said DNA fragment is derived from a microorganism belonging to the genus Escherichia .

9. A process according to any one of claims 5, 6 and 7, wherein said microorganism is a microorganism capable of biosynthesizing ATP from an ATP precursor, a phosphoryl donor and an energy donor.

10. A recombinant DNA containing a DNA fragment which is derived from a microorganism belonging to the genus Escherichia and is bearing genetic information relating to the synthesis of thiamine monophosphate and/or thiamine pyrophosphate.

11. A microorganism belonging to the genus Escherichia and which carries a recombinant DNA containing a DNA fragment which is derived from a microorganism belonging to the genus Escherichia and is bearing genetic information relating to the synthesis of thiamine monophosphate and/or thiamine pyrophosphate.

12. A process for producing thiamine monophosphate and/or thiamine pyrophosphate which comprises allowing thiamine to react with an ATP precursor, a phosphoryl donor, a catalytic amount of ATP or its precursor and an energy donor in an aqueous medium in the presence of cells, culture or treated products of a microorganism of the genus Escherichia which carries a recombinant DNA containing a DNA fragment which is derived from a microorganism belonging to the genus Escherichia and is bearing genetic information relating to the synthesis of thiamine monophosphate and/or thiamine pyrophosphate and which is capable of biosynthesizing ATP from an ATP precursor, a phosphoryl donor and an energy donor, and recovering the formed thiamine monophosphate and/or thiamine pyrophosphate from the reaction mixture.

13. A process for producing thiamine pyrophosphate which comprises allowing thiamine monophosphate to react with ATP in an aqueous medium in the presence of an enzyme source having enzyme activity to catalyze the formation of thiamine pyrophosphate from thiamine monophosphate and ATP, and recovering the formed thiamine pyrophosphate from the reaction mixture.

14. A process according to claim 13, wherein said enzyme source is thiamine monophosphate kinase.

15. A process according to claim 13, wherein said enzyme source is cells, culture or treated products of a microorganism capable of producing thiamine pyrophosphate from thiamine monophosphate and ATP.

16. A process according to claim 15, wherein said microorganism is a microorganism belonging to the genus Escherichia .

17. A process according to either of claims 15 ano 10, wherein said microorganism is a microorganism which carries a recombinant DNA containing a DNA fragment bearing genetic information relating to the synthesis of thiamine pyrophosphate.

18. A process according to claim 17, wherein said DNA fragment is derived from a microorganism belonging to the genus Esherichia .

19. A process according to any one of claims 15, 16 and 17, wherein said microorganism is a microorganism capable of biosynthesizing ATP from an ATP precursor, a phosphoryl donor and an energy donor.

20. A process according to claim 13, wherein said ATP is ATP supplied from a microorganism capable of biosynthesizing ATP from an ATP precursor, a phosphoryl donor and an energy donor.

21. A process according to claim 20, wherein said microorganism is a microorganism belonging to the genus Escherichia , Brevibacterium or Corynebacterium .

22. A process for producing thiamine pyrophosphate which comprises allowing thiamine monophosphate to react with an ATP precursor, a phosphoryl donor, a catalytic amount of ATP or its precursor and an energy donor in an aqueous medium in the presence of cells, culture or treated products of a microorganism of the genus Escherichia which carries a recombinant DNA containing a DNA fragment which is derived from a microorganism belonging to the genus Escherichia and is bearing genetic information relating to the synthesis of thiamine pyrophosphate and which is capable of biosynthesizing ATP from an ATP precursor, a phosphoryl donor and an energy donor, and recovering the formed thiamine pyrophosphate from the

reaction mixture.

# FIG.1

# FIG.2

# FIG. 3

EcoRI
CℓaI
PstI
Ptrp
Apr
pHTK111
(7.0 kb)
thiK
3.8 kb
BamHI

EcoRI
CℓaI
PstI
Ptrp
thiL
Apr
pHTL311
(5.5 kb)
4.8 kb

BamHI
T4 DNA POLYMERASE
PstI
PURIFICATION OF
3.8 kb FRAGMENT

EcoRI
T4 DNA POLYMERASE
PstI
PURIFICATION OF
4.8 kb FRAGMENT

EcoRI CℓaI
PstI
Ptrp
Apr
thiK
pTKL13
(8.6 kb)
thiL
Ptrp
CℓaI
(BamHI/EcoRI)

# FIG.4

SYNTHETIC DNA

HindⅢ    14-mer    NruI

AGCTTATG AGCTCG
     ATACTCGAGC
       Met
      10-mer

ClaI
HindⅢ
Ptrp    EcoRV
Tc'
PstI    Ap'
pKYPIO
(4.7kb)

HindⅢ
EcoRV

T4 DNA LIGASE

EcoRI
ClaI
HindⅢ
Ptrp    SacI
NruI
SacI
PstI    Ap'
BamHI
pTrS31
(4.5kb)
NruI

# FIG.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOCHIMICA ET BIOPHYSICA ACTA, vol. 258, 1972, pages 333-336, Elsevier, Amsterdam, NL; A. IWASHIMA et al.: "Conversion of thiamine to thiamine monophosphate by cell-free extracts of Escherichia coli" * Page 333, lines 10-12; page 335, lines 8-14 * | 1,4-6, 13-16 | C 12 P 17/16 C 12 N 15/54 |
| Y | IDEM | 2,3,9, 19-21 | |
| X | JOURNAL OF BIOCHEMISTRY, vol. 72, no. 5, 1972, pages 1093-110, Tokyo, JP; H. NISHINO: "Biogenesis of cocarboxylase in Escherichia coli. Partial purification and some properties of thiamine monophosphate kinase" * Page 1093, the abstract * | 13-16 | |
| Y | IDEM | 19-21 | |
| Y | CHEMICAL ABSTRACTS, vol. 101, no. 9, 27th August 1984, page 512, abstract no. 71052h, Columbus, Ohio, US; & JP-A-59 51 799 (KYOWA HAKKO KOGYO CO., LTD) 26-03-1984 * The entire abstract * | 2,3,9, 19-21 | TECHNICAL FIELDS SEARCHED (Int. Cl.5) |
| A | CHEMICAL ABSTRACTS, vol. 97, no. 15, 11th October 1982, page 370, abstract no. 123694q, Columbus, Ohio, US; N. IMAMURA et al.: "thiK and thiL loci of Escherichia coli", & J. BACTERIOL. 1982, 151(2), 708-17 * The entire abstract * | 7,8, 10-12,17, 18,22 | C 12 P C 12 N |

The present search report has been drawn up for all claims

| Place of search | Date of completion of search | Examiner |
|---|---|---|
| The Hague | 11 December 90 | RYCKEBOSCH A.O.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document